# EUROPEAN PATENT SPECIFICATION

(11) **EP 2 651 431 B1**
(45) Date of publication and mention of the grant of the patent: **07.03.2018**
(21) Application number: 11849692.6
(22) Date of filing: 16.12.2011
(51) Int. Cl.: A61K 38/23, A61N 1/36, A61N 1/372

(54) **SYSTEM AND APPARATUS FOR CONTROL OF PANCREATIC BETA CELL FUNCTION TO IMPROVE GLUCOSE HOMEOSTATIS AND INSULIN PRODUCTION**
SYSTEM UND VORRICHTUNG ZUR STEUERUNG EINER PANKREAS-BETAZELLENFUNKTION ZUR VERBESSERUNG DER GLUKOSEHOMÖOSTASE UND DER INSULINPRODUKTION
SYSTÈME ET APPAREIL POUR RÉGULER LA FONCTION DES CELLULES BÊTA PANCRÉATIQUES ET AMÉLIORER L'HOMÉOSTASIE DU GLUCOSE ET LA PRODUCTION D'INSULINE

(30) Priority: 17.12.2010 US 201061424546 P
(43) Date of publication of application: 23.10.2013
(73) Proprietor: Neural Diabetes LLC, Scottsdale, AZ 85255 (US)
(72) Inventor: PERRYMAN, Laura, Tyler, Scottsdale AZ 85255 (US)
(74) Representative: Richardt Patentanwälte PartG mbB
(86) International application number: PCT/US2011/065653
(87) International publication number: WO 2012/083259

(56) References cited:
- WO-A2-2006/087717
- WO-A2-2010/111400
- US-A1- 2007 016 262
- US-A1- 2009 312 255
- US-A1- 2010 049 274
- US-A1- 2010 268 306

## Description

### BACKGROUND OF INVENTION

The past quarter century has witnessed a dramatic increase in the prevalence in patients of a cluster of inter-related metabolic disease states, primarily caused by obesity and immune disease states, jeopardizing homeostasis and leading to the diabetic state. The incidence of diabetes, with or without obesity, has reached epidemic proportions, bringing with it impaired quality of life and life span due to serious clinical co-morbidities such as peripheral vascular and neuropathic disease, with or without pain, ulcerative skin lesions often leading to infection, gangrene, and amputation, vision loss, cardiac and renal failure and brain disorders. Without question, chronic disease associated with diabetes represents a heavy and growing burden to society in terms of both direct healthcare costs that have reached catastrophic levels and mortality rates (American Health Rankings, 2010 edition).

According to American Diabetes Association, as of 2010, 23.6 million children and adults, approximately 8% of population in the United States (US) have diabetes, and over 57 million people are clinically considered pre-diabetic in the US. According to United HealthCare, based on current trends, 52% of the US adult population could have pre-diabetes or diabetes by 2020 - up from an estimated 40% in 2010, resulting in costs estimated at $3.4 trillion for diabetes-related care over the decade from 2010 to 2020. The incident of type 2 diabetes (T2D) in adolescents has increased 10 fold from 1982 to 1994 (Pinhas-Hamiel 1996). Over 25% of obese children are considered glucose intolerant. Insulin resistance is related to inflammation and obesity induces a state of chronic inflammation. In obese states, adipose tissue secretes inflammatory agents such as cytokines. Adipose tissue macrophages alter insulin sensitivity in animal models. Obesity can be reframed as an inflammatory disease, with macrophages acting at the junction between over nutrition and inflammation.

Insulin is a peptide hormone produced by beta cells (β-cells) within the islets of Langerhans in the endocrine pancreas. Insulin promotes glucose utilization, protein synthesis, and the formation and storage of neutral lipids. Insulin is generally required for the entry of glucose into muscle. Glucose stimulates both the secretion and biosynthesis of insulin. Basal insulin secretion is normally generated to synthesize glycogen from glucose in the absence of glucose-stimulated insulin secretion.

Insulin and related insulin-like growth factors (IGF-1) give trophic support to neural tissue; their receptors are present on vanilloid transient receptor potential 1 (TRPV1) neurons; which are essential in serving to maintain neural vitality and to promoting regeneration of small sensory nerve fibers (Migdalis 1995; Sathianathan 2003; VanBuren 2005). Moreover, TRPV1 sensory neurons appear to be down regulated in pre- and post-diabetic states whereby they may fail to influence the appropriate release of calcitonin gene-related peptide (CGRP) and other neuropeptides that influence production of insulin from the beta cell (Okabayashi 1989). Reports of preclinical and clinical experiments state that exogenous administration of CGRP or induction of the endogenous release of CGRP from TRPV1 sensory neurons by the application of a TRPV 1 antagonist results in the following relevant biological responses: (1) pain signals conveyed to the central nervous system (CNS); (2) a neurogenic inflammatory response consisting of vasodilatation and edema formation, the latter not pronounced in humans; (3) insulin secretion at appropriate concentrations and (4) immunosuppression (Nagy 2004, Brain and Grant 2004; Razavi 2006). In animal models of T1D with insulinopenia, targeted expression of CGRP to β-cells or local intra-arterial administration of substance P (SP), which is co-localized with CGRP in TRPV1 sensory neurons but not as prevalent, has been reported to prevent or ameliorate diabetes (Khachatryan 1997; Razavi 2006).

In addition to T1D, β-cell dysfunction with impaired insulin regulation is also observed in subjects in the early stages of diabetes development, including impaired glucose tolerance (IGT) and obesity-related hyperinsulinemia. In obese animals, capsaicin-sensitive C-fibers containing TRPV 1 sensory neurons are markedly impaired, suggesting that intra-pancreatic neuronal release of CGRP is reduced, which would further amplify β-cell dysfunction particularly if the pancreas is maladapted to high levels of insulin (Ahren 2009). Paradoxically, the deletion or degeneration of TRPV1 sensory neurons innervating the pancreas has been reported to result in improved glucose homeostasis and insulin production (Razavi 2006; Gram 2007).

Impaired CGRP release due to TRPV1 sensory neuron pathology and/or abnormal interaction in the pathway featuring insulin production and the feedback function of the insulin receptor exhibit an inflammatory (e.g. autoimmune) state and are seen in T1D. It has been reported that increased concentrations of CGRP and other neuropeptides can prevent T1D in experimental animal models (Khachatryan 1997).

The release of CGRP to the β-cell has been reported to improve glucose and insulin homeostasis (see Gram 2005, 2007). Animal experiments have reported that sensory nerve dysfunction may contribute to hyperinsulinism, pre-diabetes initiation and progression of diabetes (Carillo 2005; Leighton and Foot 1995). Reports indicate that an imbalance in the insulin feedback control system may be "normalized" through enhancing the local supply of sufficient neuropeptides, including CGRP (Razavi 2006, Khachatryan 1997). Ablation and administration of TRPV1 antagonists have been reported to improve glucose and insulin homeostasis in subjects with pre-diabetes or T2D. See Dosch et al., United States Patent Application No. 12/478,898, published as US2009/0312255 A1.

TRPV1 sensory neurons have been shown to act as a central controller of both β-cell stress and T cell infiltration (Dosch et. al). Elimination of neurons containing TRPV1 by capsaicin or resiniferatoxin (RTX) or transection of sensory nerves innervating the pancreas and functional normalization of TRPV1 sensory neurons has the same net islet-specific outcomes: prevention of diabetes, improved glucose/insulin homeostasis, normalized insulin sensitivity and abrogation of insulitis or T1D (Szallasi 1999).

Systemic delivery of pharmaceutical agents has been the typical treatment for β-cell dysfunction and the hyperglycemia associated with IGT and diabetes; nevertheless, this approach can have limited dosing and compliance issues, and serious side effects. While non-insulin and insulin pharmacotherapies have been the hallmark in controlling hyperglycemia, there are no therapies that induce immunosuppression, and prevent/attenuate diabetes without significant risk.

Further information pertaining to the prior art can be found in WO 2006/087717 that discloses a method of treating a metabolic condition in a patient, comprising determining a target non-immediate effect of a therapy relating to treatment of a metabolic condition; and applying an electric field to an abdominal cavity of the patient in a manner designed to at least approach said target. *Inter alia,* it discloses an embodiment wherein applying the electrical signal includes configuring a frequency of an electrical signal to be between 1 and 30 Hz, an embodiment wherein applying the electrical signal includes applying pulses and configuring a pulse amplitude of the pulses to be between 2 and 15 mA, an embodiment wherein applying the electrical signal includes applying a train of biphasic pulses as well as an embodiment wherein applying the train of biphasic pulses includes setting a duration of each phase of the biphasic pulses to be between 1 and 10 ms.

WO 2010/111400 discloses a deep brain stimulation (neuromodulation) system that includes three components: an implantable pulse generator, a lead, and an extension. The pulse generator used to stimulate excitable tissue such as nerve tissue. The implantable pulse generator can be battery-powered or inductibly-powered or powered by a combination of a battery and inductibly-transmitted energy. The implantable pulse generator can be configured to deliver electrical stimulation at less than and/or greater than any of a large number of frequencies ranging from 50 Hz to 10,000 Hz. The implantable pulse generator can be configured to deliver pulse widths in the range greater than and/or less than any of a large number of pulse widths ranging from 10 µs to 3000 µs.

US 2010/268306 discloses an implantable pulse generator coupled to a lead and electrode assembly. It also teaches that electrical nerve modulation (nerve activation or inhibition) can be accomplished by applying an energy signal (pulse), at a certain frequency, amplitude, and current, to the neurons of a nerve (nerve stimulation). Where the energy pulse exceeds the activation threshold for neurons in the nerve, those neurons will depolarize, resulting in the production of action potentials. The amount of energy applied is a function of the current amplitude and pulse width duration. Activation or inhibition can be a function of the frequency, with low frequencies on the order of 1 to 50 Hz resulting in activation, and high frequencies greater than 100 Hz generally resulting in inhibition.

US 2010/049274 discloses an implantable devices that can sense, activate and/or block activity associated with one or more autonomic nerves.

### BRIEF DESCRIPTION OF THE DRAWINGS

FIG. 1 shows a diagram of a schematic view of a subject (1) with an implanted neural stimulation electrode lead (22) to stimulate the targeted sensory nerves through epidural spinal cord (20) stimulation. Also shown is a receiver (16) at the end of the electrode lead. Also shown is a typical wireless pulse generator comprising a programmer (12), a cable (14) and a antenna (18).
FIG. 2 shows a diagram of a cross-sectional view of a spinal column demonstrating a typical implantation position of the distal end of insulated electrode leads (31, 32), which terminate in electrodes (33, 34) within the epidural space (41).
FIG. 3 shows a diagram of a cross-sectional view of a spinal column demonstrating potential electrode placement at four locations: dorsal columns (81, 82, 83, 84), dorsal roots and entry zone (87, 88), dorsal root ganglia and spinal nerves (85, 86).
FIG. 4 is a diagram of a cross sectional pathway overview of a spinal column segment (201) at which an electrode lead (202) is placed to stimulate the dorsal root ganglia (203) and/or spinal nerves (210) from in the dermatome regions from spinal segments T7 to L1, which are sensory nerve fibers (204) that lead to the pancreas (206).
FIG. 5 shows a diagram of a cross sectional pathway overview, as in FIG. 4, but where the electrode lead (302) is placed to stimulate the splenic nerve.
FIG. 6 shows a diagram of the various dermatome levels for nerve fibers to various tissues of the typical body of a subject (400). The viscera region is innervated by most of the dermatomes ranging from T7 to L1 (401).
FIG. 7 shows various configurations for an electrode lead array. Shown are (a) four electrode leads with 3 mm spacing (501), (b) four electrode leads with spacing less than 3 mm (502), (c) 8 electrode leads with regular spacing on the lead (503) four electrode leads with spacing that includes electrodes at the tip, with remote anodes to create a wide area of volume conduction (504), (d) electrode pads (505) and embedded paddle electrode leads (506).
FIG. 8 shows a neural stimulator device powered by an implanted pulse generator (IPG).
FIG. 9 is a schematic diagram of a closed-loop controller, which includes sensing of physiological parameters and adjusting stimulation parameters.
FIG. 10 shows a cartoon of the feedback loop between a small sensory nerve fiber (SSNF) ending containing TRPV1 sensory neurons (e.g. C-fibers) secreting neurogenic peptides and beta cells stimulating insulin action.
FIG. 11 shows the effect of neural stimulation upon abdominal blood flow in the rat model for diabetes: (A, top panel) area under the curve (AUC) in Zucker Lean (ZL) and Zucker Fatty (ZF) rats at the indicated frequencies (5 Hz or 100 Hz) and pulse durations (0.2 ms and 1 ms); (B, bottom panel) time course of spinal cord stimulation of rats at the indicated frequencies (5 Hz, increase in blood flow, or 100 Hz, decrease in blood flow) and pulse duration of 1 ms.
FIG. 12 spinal cord stimulation (SCS) shows the insulin response (amount secreted) resulting from electrical neural stimulation treatment on Zucker Fatty (ZF) rats.
FIG. 13 shows photomicrographs of PGP9.5 stained pancreatic islet cells of Zucker Lean (ZL) and Zucker Fatty (ZF) rats post neural innervation.
FIG. 14 shows photomicrographs of PGP9.5 and glucagon stained pancreatic islet cells of Zucker Lean (ZL) untreated control rat (top panel) and a ZL rat post neural innervations (bottom panel). Green: insulin (beta cells); White: glucagon (alpha cells); Red: PGP 9.5 (neurons); Scale bar: 75 um.
FIG. 15 shows photomicrographs of PGP9.5 and glucagon stained pancreatic islet cells of Zucker Fatty (ZF) untreated control rat (top panel) and a ZF rat post neural innervations (bottom panel). Green: insulin (beta cells); White: glucagon (alpha cells); Red: PGP9.5.

### SUMMARY OF THE INVENTION

The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention. Provided herein are methods, systems and apparatuses for preserving, restoring or affecting pancreatic beta cell function in a subject. These methods include electrically stimulating C-afferent sensory nerve fibers innervating pancreatic beta cells in a subject, in which the electrical stimulation modulates a secretion of calcitonin gene-related peptide (CGRP) from the C-afferent sensory nerve fibers; determining a level of a biomarker in the subject and repeating the electrical stimulation as a function of the level of the biomarker.

The methods, systems and apparatuses include electrical stimulation carried out via at least one anode electrode and one or more cathode electrodes, the at least one anode electrode serving as a reference electrode. The two or more electrodes are positioned in the subject proximal to nerve tissue, including one or more of: epidural spinal cord column at any of vertebral segments T7 to L1, dorsal root or dorsal root entry zone at any of vertebral segments T7 to L1, spinal nerve bundles leaving at any of the vertebral segments T7 to L1, dorsal root ganglia bundles leaving at any of the vertebral segments T7 to L1, peripheral nerves innervating the endocrine pancreas beta cells, abdominal nerves or their cutaneous branches, a surface of endocrine pancreas, or combinations thereof. The two or more electrodes are placed ipsilaterally or bilaterally, preferably in the epidural space. The electrical stimulation can be carried out using multiple electrode pairs, either simultaneously or sequentially.

The methods, systems and apparatuses include electrical stimulation that is effected wirelessly.

The methods, systems and apparatuses include electrical stimulation that is carried out at the following parameters: a pulse width from 20 µsec to 1 ms, a frequency from 1 Hertz (Hz) to 10,000 Hz, and power amplitude from 0.2 to 14 Volts (V) or 0.1 to 20 milliamps (mA). The frequency of the electrical stimulation is between 5 and 10,000 Hz. The frequency of said electrical stimulation is between 1 and 50 Hz (resulting in enhancement of the secretion) and, optionally, the pulse width is in the range of about 250 to about 450 microseconds. The frequency of the electrical stimulation is between 60 and 10,000 Hz (resulting in the inhibition of the secretion) and, optionally, the pulse width is in the range of about 500 to about 1000 microseconds.

The methods are directed to a subject who suffers from beta cell dysfunction or impairment, is pre-diabetic or suffers from obesity-related inflammation, suffers from impaired glucose tolerance (IGT), suffers from diabetes mellitus, suffers from Type I diabetes, suffers from Type II diabetes or suffers from diabetes insipidus.

The methods are performed in which the subject needs not adopt a lifestyle change.

The methods are performed in which the subject effects the step of repeating the electrical stimulation as a function of the level of a biomarker or where the step is effected automatically. The biomarker includes, but is not limited to, any one or more of insulin, glucose, CGRP, H1Abc, abdominal skin blood flow, abdominal skin temperature, and abdominal muscle electrical activity.

The method is performed in which the biomarker is insulin and the subject is a male subject and the level is below about 8.8 µIU/mL or the subject is a female subject and the level is below about 8.4 µIU/mL; and the electrical stimulation is carried out to excite the C-afferent sensory nerve fibers innervating pancreatic beta cells. The method is performed in which the biomarker is insulin and the subject is a male subject and the level is above about 8.8 µIU/mL or the subject is a female subject and the level is above about 8.4 µIU/mL; and said electrical stimulation is carried out to inhibit the C-afferent sensory nerve fibers innervating pancreatic beta cells. The method is performed in which the biomarker is glucose and the level is above about 120 mg/dL, and the electrical stimulation is carried out to excite the C-afferent sensory nerve fibers innervating pancreatic beta cells. The method is performed in which the biomarker is glucose and the level is below about 100 mg/dL, and the electrical stimulation is carried out to inhibit said C-afferent sensory nerve fibers innervating pancreatic beta cells.

"C-afferent sensory nerve fibers" means unmyelinated postganglionic fibers of the autonomic nervous system, also the unmyelinated fibers at the dorsal roots and at free nerve endings, that convey sensory impulses from the periphery to the central nervous system.

"Subject" means any animal, such as a human, with an insulin-producing organ, such as an endocrine pancreas.

"Pancreatic beta cells" means insulin-producing cells situated in the islets of Langerhans.

"Electrical stimulation" means the application of electrical current to stimulate nerves.

"Biomarker" means any physiological indicating species produced by a subject. Examples of biomarkers include, but are not limited to, insulin, glucose, tissue CGRP, abdominal skin blood flow, abdominal skin temperature, and abdominal muscle electrical activity.

"Electrode" means an electrical conductor used to make contact with a nonmetallic part of a circuit. An electrode can be an anode or a cathode. An electrode pair means two electrodes: one anode and one cathode. Configurations of multiple electrodes can be multiple electrode pairs or one or more anode or cathode with any number of electrodes of the reverse polarity.

"Epidural spinal cord column" means the space superficial to the dura matter that exists between it and the internal surfaces of the vertebral bones and their supporting ligamentous structures of the spine.

"Dorsal root or dorsal root entry zone" means the posterior root that is the afferent sensory root of a spinal nerve.

"Dorsal ganglia" means the nerve structure at the distal end of the dorsal root, which contains the neuron cell bodies of the nerve fibers conveyed by the root.

"Spinal nerve bundles" means nerves within the spinal cord that are grouped together.

"Peripheral nerves" means nerves and ganglia outside of the brain and spinal cord.

"Pre-diabetic" means a condition thought to be a precursor of adult-onset diabetes mellitus, marked by carbohydrate intolerance or other symptoms of the disease.

"Impaired glucose tolerance (IGT)" means a pre-diabetic state of dysglycemia that is associated with insulin resistance and increased risk of cardiovascular pathology.

"Diabetes mellitus" means diabetic states that include T1D, T2D and gestational diabetes.

"Type I diabetes (T1D)" means a condition characterized by loss of the insulin-producing beta cells of the islets of Langerhans in the pancreas leading to insulin deficiency. This type of diabetes can be further classified as immune-mediated or idiopathic. Type 1 diabetes can affect children or adults but was traditionally termed "juvenile diabetes" because it represents a majority of the diabetes cases in children.

"Diabetes insipidus" means a condition characterized by excessive thirst and excretion of large amounts of severely diluted urine, with reduction of fluid intake having no effect on the latter.

"Type II diabetes (T2D)" means a condition characterized by insulin resistance which may be combined with relatively reduced insulin secretion.

"Lifestyle change" means changes in diet, exercise, nutraceutical and pharmaceutical regimens.

### DETAILED DESCRIPTION OF THE EMBODIMENTS

Provided herein are methods, systems and apparatuses to provide electrical neural stimulation of neurons containing the TRPV 1 receptor, small sensory nerve fibers of other TRPV sub-families, and autonomic nerve fibers to control the production of insulin from endocrine pancreatic beta cells. The disclosed examples are related to the excitation or inhibition of the small sensory nerves, preferentially C-afferent fibers, by electrical neural stimulation of the nerve fibers that innervate the endocrine pancreas and act upon pancreatic beta cells. Such methods, systems and apparatuses can be used to treat pre-diabetic or diabetic states in a subject. Examples disclosed herein allow stimulation of neurons containing the vanilloid transient receptor potential 1 (TRPV1) receptor, many of which release a neuropeptide, calcitonin gene-related peptide (CGRP), which in turn influences the production of insulin from pancreatic beta cells. Embodiments of the invention further include a closed or open loop feedback control system in which biomarker levels are monitored and used to regulate the applied neural stimulation. Embodiments of the invention also include an implantable or external neural stimulation device.

While not wishing to be held by theory, examples are based on observations that electrical stimulation of the dorsal root ganglia sensory nerve fiber bundles reverses insulin resistance. Thus, in insulinopenic states, purportedly due to immune and/or endocrine dysfunction and associated with down-regulation of insulin receptors, CGRP (with SP) release induced by electrical stimulation of specific neural tissue can serve as insulin "replacement therapy." CGRP can therefore be used therapeutically in terms of improving the local immunoregulatory state of the endocrine pancreas, pancreatic neurogenic vasodilatation (blood flow) and balancing an abnormal functional circuit comprised of low insulin production-low activation of TRPV1 sensory neurons-low release of CGRP. This concept also supports a view of a neuro-immune-endocrine link in β-cell function and the role for insulin-responsive TRPV1 sensory neurons in β-cell function and diabetes pathoetiology.

Consequently, there appears to be a local feedback interaction between β-cells and the TRPV1 sensory neurons innervating the islets with nerve terminals responding to insulin by release of neuropeptides that sustain β-cell physiology in an optimal range. Normally this interaction is in "balance" but in T1D animal models, as well as in the pre-diabetic obese and T2D models, hypofunction of TRPV1 unbalances the feedback, with β-cell stress due to hyperinsulinism, insulin resistance, and infiltration by T-cell pools independently generated. Removing TRPV1 sensory neurons (e.g., desensitization) leads to elimination of the unbalanced pathogenic interaction whereas administering neuropeptides exogenously (or by sensitization of TRPV1 neurons) may renormalize the interaction. Consequently, suppressed neuropeptide release due to impaired TRPV1 sensory neurons and/or to abnormal β-cell function including down-regulation of insulin receptors on TRPV1 sensory neurons can be addressed by two approaches. One approach is the desensitization, removal or inhibition of TRPV1 sensory neurons. Another approach is the sensitization or excitation of TRPV1 sensory neurons. Both treatment options can re-balance the interaction between the β-cell and the TRPV1 sensory neuron depending on the state of the subject's disease state. Further, improved local insulin production induced by release of CGRP from TRPV1 sensory neurons can restore function of the normalized feedback and glucose-insulin homeostasis by promoting insulinotrophic action on sensory nerves in subjects where production of insulin is severely impaired.

Examples include the control of glucose homeostasis by stimulation of TRPV1 sensory neurons that innervate the endocrine pancreas. Electrical neural stimulation can be performed by directing the electrical stimulation at one or more anatomical sites including, but not limited to, 1) epidural spinal cord column, which can include the area at vertebrae segments T7 to L1 (and can include branches that innervate the endocrine pancreas beta cells); 2) dorsal root and dorsal root entry zone, which can include the area at vertebrae segments T7 to L1; 3) spinal nerve bundles, which can include the area leaving the vertebrae segments T7 to L1; 4) dorsal root ganglia bundles, which can include the area leaving the vertebrae segments T7 to L1; 5) peripheral nerves, such as the splenic nerve which innervate the endocrine pancreas beta cells; 6) abdominal nerves or their cutaneous branches (which can be stimulated by TENS or other external stimulation); and 7) directly at the surface of the endocrine pancreas. Electrical neural stimulation may be in more than one area simultaneously or sequentially, ipsilaterally or bilaterally.

While not wishing to be held by theory, in an aspect examples are directed to methods, systems and apparatuses that activate/deactivate TRPV1 sensory neurons associated with pancreatic beta cell function by using charge-balanced voltage or current controlled electrical pulses originating from the electrical neural stimulation volume conduction of electrode leads. The TRPV1 sensory neuron can be stimulated at a frequency range from 1 to 10, 20 or 50 Hz. This frequency range often results in excitation of the TRPV1 sensory neurons. Such excitation can increase β-cell activity and promote insulin release. The preferred frequency for neural excitation can be 5 Hz. For inhibition of the TRPV1 sensory neuron, which can mimic the effects of ablation or receptor blocking, a frequency of 60 Hz or greater can be applied. Such inhibition can balance the insulin-dependent TRPV1 neuronal feedback system, thereby increasing insulin release. The frequency range for neural inhibition can be from 60 to 10,000 Hz. The preferred frequency for neural inhibition can be 100 Hz.

These and other electrical stimulation parameters used to stimulate TRPV1 sensory neurons can differ from those commonly used clinically for control of inflammatory and neuropathic pain and peripheral vascular disease. Stimulation parameters, in addition to frequency, that can be modulated include, but are not limited to, duty cycle, duration, waveform shape, amplitude, voltage, and magnitude. In an embodiment, the pulse width is in the range of 20 microseconds (µsec) to 1 millisecond (ms). The pulse width can be 250 µsec to 450 µsec, which can result in excitation of the TRPV1 sensory neuron. The pulse widths can be 500 µsec to 1 ms, which can result in inhibition of the sensory neuron. The power amplitudes can be from 0.2 to 14 Volts, or 0.1 to 20 mA, depending on whether the power is voltage or current-driven.

In another embodiment of the invention, one or more biomarker levels in the subject are monitored and the information resulting from such monitoring is used to determine subsequent delivery of electrical stimulation. The biomarker can be insulin or glucose. Normal levels of insulin are about 8.8 uIU/mL in male subjects and about 8.8 uIU/mL in female subjects. Normal levels of blood glucose are about 100-120 mg/dL. Consequently, in certain embodiments of the invention, the C-afferent sensory nerve fibers innervating pancreatic beta cells are either excited or inhibited to promote glucose homeostasis in response to abnormal biomarker levels. The biomarker can also be abdominal skin blood flow, abdominal skin temperature, or abdominal muscle electrical activity. Abdominal muscle electrical activity can be monitored by electromyography (EMG). Subsequent delivery of electrical stimulation can be performed by open loop control, whereby the subject is notified to begin, end or adjust parameters of the stimulation. Subsequent delivery of electrical stimulation can be performed by closed loop control, by an apparatus comparing sensed physiological values to historic or normative values, and automatically adjusting the stimulation output accordingly.

In further examples, the methods, systems and apparatuses disclosed herein are used to protect the endocrine pancreas against abnormal immune-cell accumulation or inflammation (insulitis, i.e. Type I Diabetes (T1D)). While not wishing to be held by theory, impaired CGRP release due to TRPV1 sensory neuron pathology and/or abnormal interaction in the pathway featuring insulin production/insulin receptor function and CGRP release favor an inflammatory state and, hence, T1D becomes a model of immune dysregulation, due to the early onset of sensory nerve impairment leading to inflammatory destruction of insulin-producing β-cells and to insulin deficiency and hyperglycemia. Increased concentrations of neuropeptides like CGRP prevent Type I diabetes in experimental animal models. Endogenous produced CGRP is therefore used therapeutically in terms of improving the local immunoregulatory state of the endocrine pancreas, pancreatic neurogenic vasodilatation (blood flow) and balancing an abnormal functional circuit comprised of low insulin production-low activation of TRPV1 sensory neurons-low release of CGRP.

In further examples, the methods, systems and apparatuses disclosed herein are used for the treatment of pre-diabetic state and diabetes. While not wishing to be held by theory, sensitization of TRPV1 neurons stimulates insulin action and improves glucose and insulin homeostasis. Consequently, in various examples, pre-diabetes, T1D, Type II (T2D), diabetes mellitus, diabetes, diabetes insipidus and beta cell deficiency syndrome can be treated.

Typically, pre-diabetes and diabetes treatments include lifestyle modifications. Lifestyle modifications include changes in diet, exercise, nutraceutical and pharmaceutical regimens. In an example, the methods of the invention further include lifestyle modifications. In another example, the methods of the invention are applied without the need for lifestyle modifications.

Also provided are systems and apparatuses for stimulation of TRPV1 sensory neurons that innervate the endocrine pancreas.

The system can include an electrode lead or a multiple electrode lead array as disclosed elsewhere in this application.

The system can further include means for electrical neural stimulation in an open loop format. For example, the system can alert the subject to a change in glucose homeostasis, thereby allowing the subject to choose whether to initiate another electrical neural stimulation. The alert can be triggered by a sensor that detects a biomarker level achieving a specified threshold. In another example, the device can be programmed to stimulate certain neural tissues at predetermined intervals, such as to maintain hormone levels at a certain concentration.

The system can further include means for electrical neural stimulation in a closed loop format. The system can include a feedback sensor. The feedback sensor can collect information on biomarker levels and transmit to a controller to compare measured levels to desired ranges. If outside the desired or threshold range, the feedback controller can initiate adjustments to parameter settings of the electrical stimulation. The efficacy of electrical stimulation can be evaluated so that the parameter settings can be adjusted to improve the response. For example, the duration of a pulse of stimulation can be adjusted automatically to improve response.

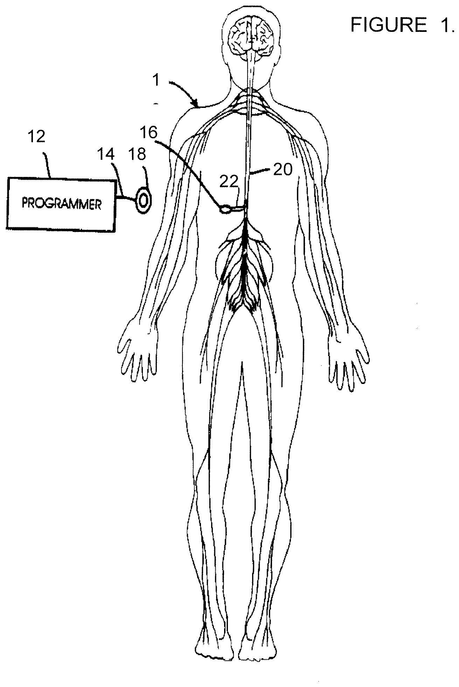

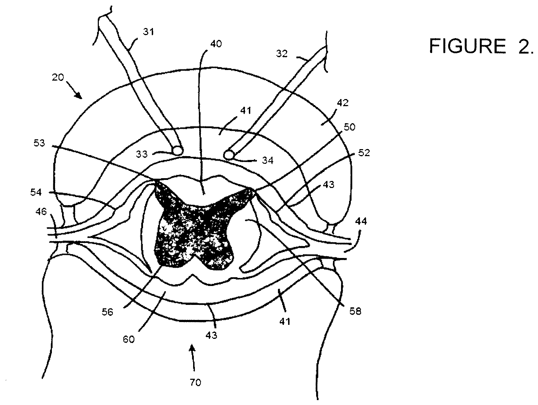

FIG. 1 shows a schematic view of a subject **10** having an implant of a neural stimulation electrode lead to stimulate the targeted sensory nerves through epidural spinal cord stimulation **20.** The system can employ an implantable or external pulse generator **16** to produce a number of independent stimulation pulses which are sent to the spinal cord **20** by insulated lead **22** or wirelessly which has two or more electrodes **33** and **34** (FIG. **2**). The implantable pulse generator **16** can have an internal battery and pulse-generating electrical components. The system can further comprise a radiofrequency antenna **18,** which can be connected to an external programmer **12** by an extension **14.** Alternatively, the pulse generator **16** can electrically process incoming radiofrequency signals from the antenna, and produce electrical pulses sequentially, without aid of a battery.

Electrodes can be placed near neural tissue. FIG. **2** is a diagram of a cross-sectional view of a subject spinal column **20** of a subject showing an embodiment where the implantation position of the distal end of insulated electrode leads **31** and **32,** which terminate in electrodes **33** and **34** within the epidural space **41.** Electrodes can be made of a platinum/iridium compound. The electrodes are shown relative to the subdural space **60** filled with cerebrospinal fluid (CSF), bony vertebral body **70,** vertebral arch **42,** and dura mater **43.** The spinal cord includes gray matter **56** and white matter, for example, dorsal columns **40** and dorsolateral columns **58.** At the dorsal tips of the gray matter are the dorsal roots **50** and **53,** which are axons, originating from cell bodies in the dorsal root ganglia, **52** and **54.** These same cell bodies have sensory endings in tissue, and their axons pass along spinal nerves **44** or **46.** Stimulation pulses can be applied to at least one of electrodes **33** and **34** (which typically are cathodes); while at least one anode can be used for electrical return paths at other epidural space **41** locations. Models of electric fields with spinal cord stimulation (cf. Jan Holsheimer et al.), and clinical experience suggest that not only are axons in the dorsal columns **40** excited, but so are axons in the dorsal roots **50** and **53,** and possibly also axons near to dorsal gray matter several millimeters away.

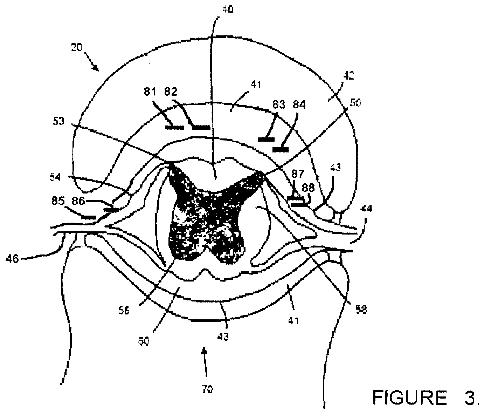

Electrodes can be placed at one or more sites. FIG. **3** is a diagram of a cross-sectional view of a spinal column showing embodiments of electrode placement at different implantation sites to stimulate dorsal columns, dorsal roots and entry zone, dorsal root ganglia and/or spinal nerves. The diagram shows the spinal cord **20** of the subject relative to implantation sites of the electrodes which are useful to therapeutically control TRPV1 sensory neurons: 1) electrodes **81** and **82** for epidural spinal cord stimulation at segments T7 to L1 (sites with relatively significant proportions of branches of TRPV1 neurons innervating the pancreas); 2) electrodes **83** and **84** for dorsal root and dorsal root entry zone stimulation at segments T7 to L1; 3) electrodes **85** and **86** spinal nerve stimulation of nerves T7 to L1); 4) electrodes **87** and **88** for dorsal root ganglia stimulation of T7 to L1. Electrodes for therapeutic stimulation can also be placed at: 5) peripheral nerves innervating the pancreas; 6) abdominal nerves or their cutaneous branches; and/or 7) the surface of the pancreas. Two or more electrodes can be used, at least one of which is a cathode (negative) and at least one of which is an anode (positive). One or more of the electrodes used can be at spinal levels. In the case of anode(s), the electrode can be distant. Pulses may be current or voltage controlled. The pulses can be charge-balanced for safety. Electrodes can be placed in the epidural space, outside the dura, or subdurally. Electrodes may be placed nearby, outside the perineural sheath, or inside and along the nerve fibers of peripheral nerves.

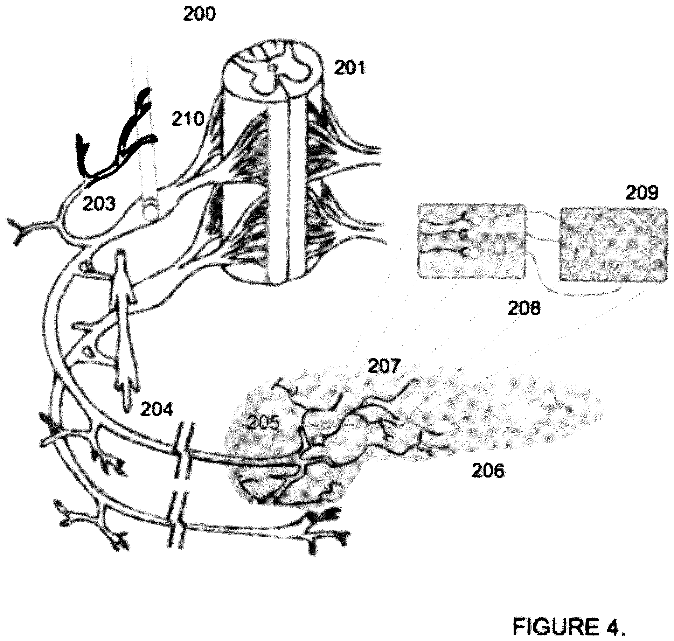

The electrodes can be placed near the dorsal root ganglia and/or the spinal nerves. **FIG. 4** is a diagram of a cross sectional pathway overview **200** of an exemplary spinal column segment **201** showing positioning electrode lead **202** to stimulate the dorsal root ganglia **203** and/or spinal nerves **210** by placement perpendicularly with the nerve bundle leaving the particular dermatome. The electrode lead placement in FIG. **4** can stimulate TRPV1 sensory neurons via the dorsal root ganglia in the dermatome regions from spinal segments T7 to L1, which are sensory nerve fibers **204** that lead to the pancreas **206.** Electrodes can also be placed at the: 1) peripheral nerves innervating the pancreas **204;** 2) abdominal nerves or their cutaneous branches; and/or 3) the surface of the pancreas **206.** While pairs of electrodes **202** are shown in Fig. **4****,** stimulation can be effected with two or more electrodes, at least one of which is a cathode (negative) and at least one of which is an anode (positive). One or more of the electrode leads used can be placed at different spinal levels or even distant in the case of anode(s).

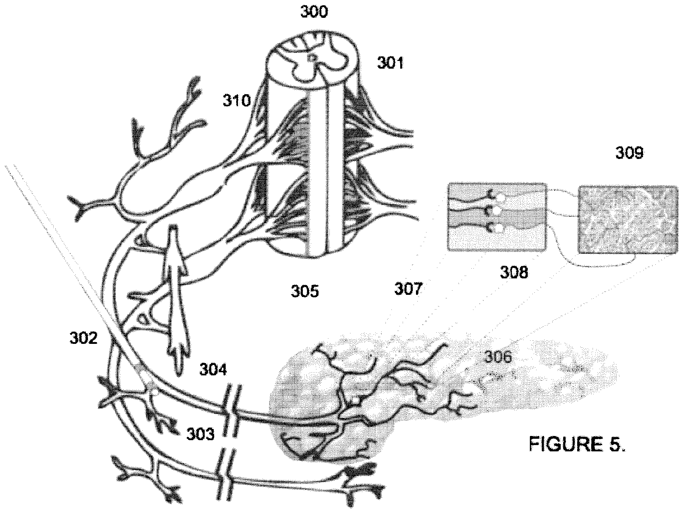

The splenic nerve can be a site of electrode placement. FIG. **5** is a cross sectional pathway overview **300** of a subject spinal column segment **301** showing an electrode lead **302** placed to stimulate the splenic nerve **303** by placement in parallel with the nerve bundle **304** leaving the particular dermatome level spinal segments **310.**

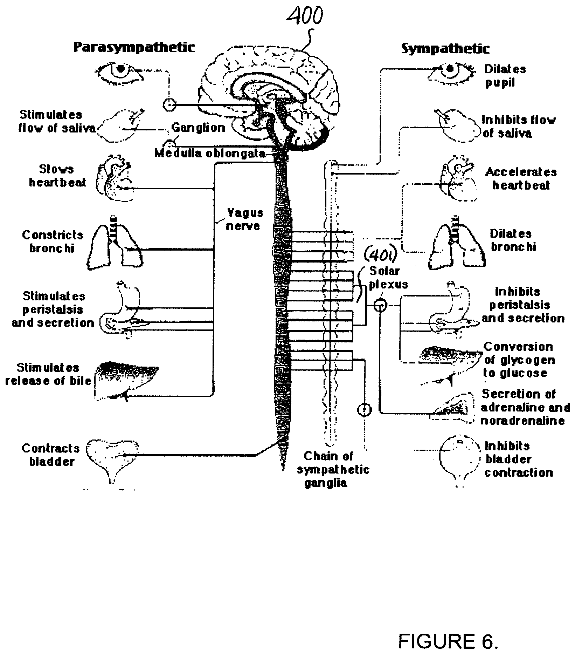

FIG. **6** shows the various dermatome levels for nerve fibers to various tissue of the typical subject **400.** The viscera region is innervated by most of the dermatomes ranging from T7 to L1

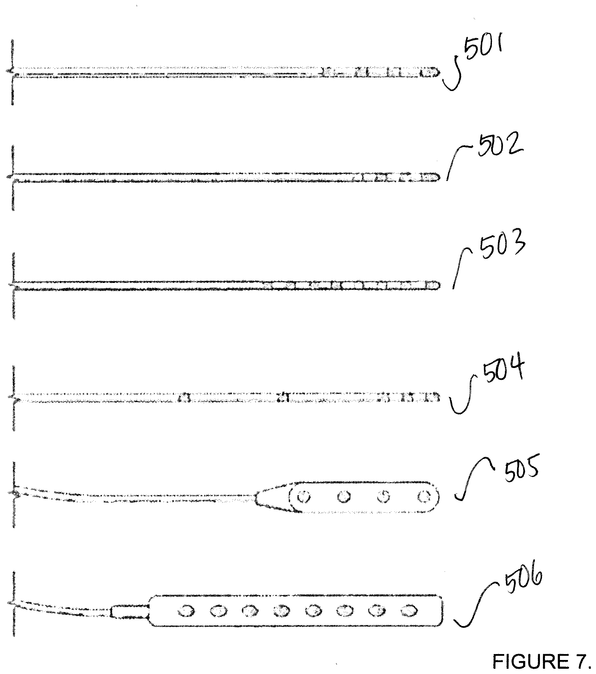

Electrode types and electrode spacing can be modified in suitable embodiments. FIG. **7** shows various optional configurations for the electrode lead array that can be placed in the epidural space, directly on the dorsal root ganglia, in the region of the splenic nerve, or other location along the neuronal pathway from the dorsal column to the pancreas originating from dermatomes in the region of T7 to L1. Typically, four electrode leads are sufficient for peripheral nerve stimulation placements **501.** The spacing of these electrodes can be about 3 mm, as shown **501,** however studies have shown that targeting of the nerve bundle can be improved by electrode spacing that is less than 3 mm **502.** In other examples, such as for greater range of coverage in epidural space placements, leads with 8 electrodes **503** or more can be used to cross over multiple dermatome levels. In other embodiments **504,** spacing can include electrodes at the tip, with remote anodes to create a wide area of volume conduction. In yet another example, the electrode pads can be placed in a configuration that eliminates the 360 degree electrode wrapping around the lead **503** and places the electrodes embedded in a lead assembly where placement must be manually inserted to lie against the spinal column within the lead unilateral volume conduction area **505.** Embedded paddle electrode leads can have a multitude of electrode pads **506.**

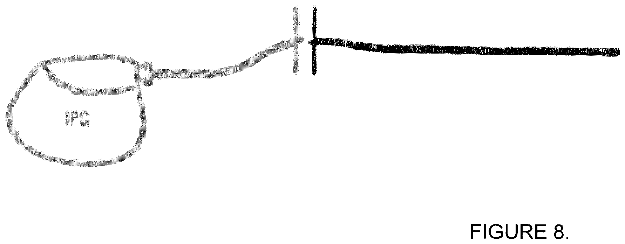

In some embodiments, the invention further comprises an implanted pulse generator (IPG) that can be used for open- or closed-loop feedback. FIG. **8** is a diagram showing placement of an IPG. The IPG can be located subcutaneously in the abdomen or lower back region and tunneled by extension wire to the electrode lead at the therapeutic treatment area. Alternatively, pulse information can be received for this treatment protocol by an external pulse generator sending information to an imbedded antenna receiver which interprets and correlates the instruction set to provide the electrode lead with the appropriate therapeutic parameter sets.

The system and apparatus can further include a controller. The controller can generate excitatory or inhibitory electrical stimulation to TRPV1 sensory neurons to alleviate the imbalance between insulin production from the β-cell-CGRP release. The controller can communicate with one or more sensors to detect biomarker levels as disclosed herein.

The controller can comprise one or more transmitters and receivers in communication with sensors and the electrical stimulation device such as the pulse generator. The controller can send the signals to increase or decrease stimulation until glucose homeostasis is achieved. The stimulus parameters include, without limitation, amplitude, pulse duration, duty cycle, pulse width/frequency, and polarity of electrodes on the lead. The controller can include a microprocessor that can instruct the system to produce an exciting or inhibiting stimulation signal or to cease electrical stimulation. The microprocessor can be programmed with preselected stimulus parameters. The receivers receive signals from the sensors, process signals to be analyzed by the controller and store the signals in a data storage and/or pre-processor area, such as a dynamic random access memory (DRAM). Sensors sense various biomarker levels to determine, for example, whether there has been sufficient glucose homeostasis control in the neuroendocrine system.

The controller can be an external device or an implantable device. In certain embodiments, it can provide signals to a subject who is experiencing an unexpected event. The controller can be programmed for either automatic or manual operation. The controller can have one or more conventional glucose sensors. Upon detection of a hormonal irregularity, the controller can automatically begin treatment of the subject by regulating hormone levels through electrical stimulation. In another example, the subject can manually activate the controller. The activation can begin or adjust regulation of CGRP levels. The activation can regulate insulin homeostasis. A positive physiological response, e.g. a physiological response that trends to the "normal" range, can be used as an indication that the electrical stimulation is effective in producing glucose homeostasis.

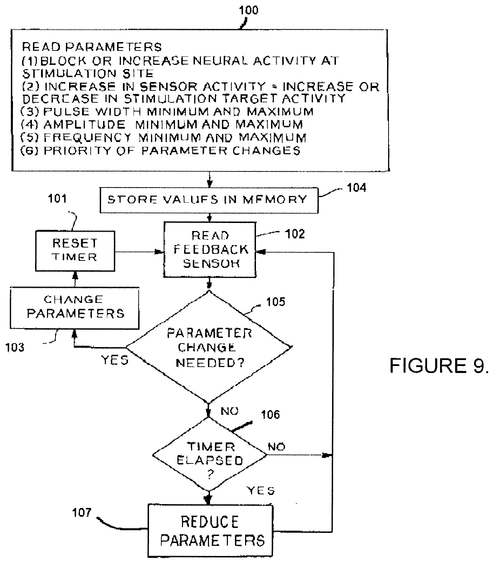

In some embodiments, the invention further comprises a closed-loop controller. **FIG. 9** is a schematic diagram of a closed-loop controller, which includes sensing of biomarker levels and adjusting stimulation parameters. The closed-loop controller generates excitatory or inhibitory electrical stimulation to TRPV1 neurons alleviating the imbalance between insulin production from the β-cell-CGRP production. The controller utilizes one or more sensors to detect the biomarker level. The controller compares sensed biomarker levels (data) **102** to stored historic or normative levels **104,** and adjusts the stimulation output accordingly. Physiological normative data, past data from this subject, and ranges of stimulation parameters can be saved in storage element **100.** Once sensed data is acquired **102,** a decision can be made **105,** optionally based on priorities, whether stimulation must be initiated or altered to affect this parameter. The controller can effect changes to parameters or to anatomical locations of stimulation as required **103.** The controller can effect these changes independent of altering the timing element **101** of the electrical stimulation. If the decision to change a parameter or location of stimulation is negative, the timer will be checked **106,** and either the timer will be reset, or parameters of stimulation in general can be reduced **107,** but not below *α priori* limits. The controller can consult with two or more sensed physiological values before making decisions.

### EXAMPLE 1

### The biological feedback loop between SSNF and beta cells

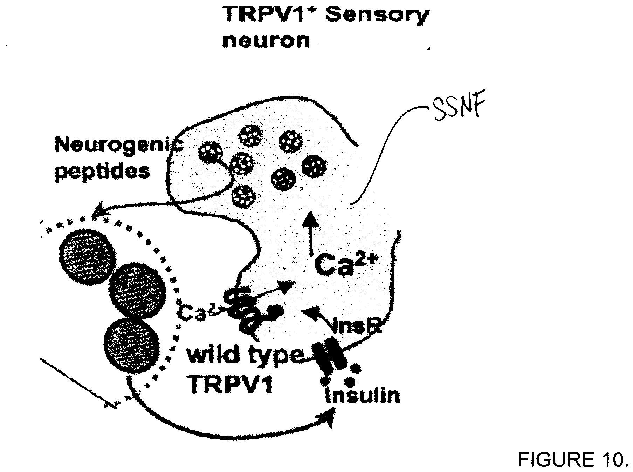

The biological feedback loop between a small sensory nerve fiber (SSNF) ending containing TRPV1 sensory neurons (e.g. C-fibers) secreting neuropeptides and beta cells stimulating insulin action can be controlled by electrical stimulation. FIG. **10** shows a cartoon of a small sensory nerve fiber ending containing TRPV1 sensory neurons (e.g. C-fibers) when modulated by electrical neural stimulation techniques by methods disclosed herein act on the dorsal root ganglia through volume conduction from a dorsal column placement of an electrode lead. The neuropeptide CGRP, among others, are released from the activated TRPV1 sensory neuron terminal. The neuropeptide acts on the beta cell stimulating insulin action through an inflammatory response action. spinal cord stimulated treated animals with excitatory protocols show a marked effect on insulin production. This closed loop system can also be inhibited to cut off the production of insulin from the beta cell. Insulin released from the beta cell acts on the insulin receptor located on the TRPV1 sensory neuron terminal. The influx of insulin into the sensory nerve cells up regulates the TRPV1 receptor channel and an influx of calcium enters the cell, which leads to the release of CGRP neuropeptide from the cell, closing the loop to act on the beta cell invoking insulin release. Electrical neural stimulation parameters are set in this disclosure to both stimulate the cycle of release of insulin and inhibit the cycle with blocking parameter settings.

### EXAMPLE 2

### Materials and Methods

All experimental procedures comply with the guidelines of the National Institute of Health Guide for the Care and Use of Laboratory Animals. Six adult male, Zucker lean (250-300 g) and nine Zucker obese (450-550 g) animals (Charles River, MA) were deprived of food for 24 hours before the experiments but allowed free access to water. Non-steroidal analgesic antiinflammatory (Meloxicam @ 1 mg/kg, SC), antibiotic (Clavamox @ 15 mg/kg, PO [approx 0.1 ml/rat]), and pain reliever (buprenorphine @ 0.04 mg/kg, SC) were administered 30 minutes prior to induction. During the experiments, all animals were anesthetized with isoflurene and artificially ventilated (Harvard Rodent Ventilator) at 2% ventilation with oxygen. All surgical procedures were performed under aseptic conditions. Body temperature was maintained between 36 and 38 °C with a heating pad. An acute surgical study was performed for up to 8 hours. Mean arterial blood pressure and heart rate (HR) were continuously monitored with a rat tail cuff monitor (Kent Scientific). Rectal temperature was also monitored and maintained between 36 to 38°C with a heating pad. If any of these variables fell below physiological levels the experiments were terminated. A fall in blood pressure below 80-90 mmHg resulted in rapid deterioration of the antidromically evoked C-fiber excitation. The rats were immobilized in a modified stereotactic spinal apparatus (David Kopf Instruments Inc., Tujunga, CA) for the duration of the experiments.

### Placement of Epidural Neural Stimulator

A mid-dorsal skin incision was made from L1-L2. The fascia and muscles at the appropriate vertebral level was separated. In all rats, with the aid of an operating microscope, a laminotomy (using a rongeur to clip the process until free and open a gap to 3 mm in diameter) was performed to gain access to the dorsal surface of the lower thoracic lumbar segments of the spinal cord at the L1-L2 vertebrae levels. Small cotton swabs were used to dry the area.

Stimulation electrodes were made from platinum wire (Heraus). The stimulation electrode was placed at the level of the 10th to 11th thoracic (T10-T11) vertebrae directly. The implantable stimulation device has an electrode diameter of 1 mm and in the range of 6 mm long. It is made of inert materials including platinum-iridium electrodes, a silicon microelectronic chip, and a biocompatible silicone coating (Specialty Coatings). When actuated the implanted device produces small rectangular pulses of a neurostimulating current. An electrical stimulator (AM Systems Inc. Model 2000) was connected by wire to an autoclaved introducer electrode and a reference ground silver chloride electrode was inserted subcutaneously on a shaved portion of the mid-back region. The electrodes were secured in place with suture 4-0 monofilament absorbable (Monocryl) suture to anchor device.

Spinal cord stimulation (SCS) was applied at the epidural surface of T10-T11 vertebrae spinal location in the center of the cord. SCS has been shown to increase vasodilation in the skin through release of CGRP from the afferent fibers in the dorsal roots (Tanaka et al 2004). Muscle twitch threshold and threshold under epidural stimulation was determined. Continuous epidural electrical stimulation was delivered at 5 Hz or 100 Hz at 200µs and 1 ms durations. The stimulation intensity ranged from 40 µA to a maximum of 120 µA.

The Motor Threshold (MT) stimulus intensity was determined in each animal at 5 Hz, 0.2 ms by slowing increasing the SCS currents amplitudes from zero until a clear abdominal contraction was apparent. SCS was performed for 2 minutes at 100% MT, 110% MT and 120% MT for each stimulation parameter setting, 5 Hz, 0.2 ms, 5 Hz, 1 ms, 100 Hz, 0.2 ms and 100 Hz, 1 ms. EMG waveform results of SSNF stimulation with the pulsed with biphasic, 0.2 ms, 5 Hz repetition rate. Waveforms were recorded at a current level that produced C-fiber excitation onset.

### Measurement of blood flow

Local blood flow was measured in the dorsal skin of the rats with a Moor Instruments laser Doppler blood flow meter. The dorsal skin was shaved and depilated with a commercial depilatory cream (Nair) and cleaned with an alcohol swab to remove any dead, dry or impermeable layers of skin that could impair the Doppler measurements. Skin sites were marked out on the dorsal skin according to a balanced site pattern with two sites for each measurement on the right side of the abdomen of the rat corresponding to the location of the pancreas. Laser probes were adhered to the skin with an adhesive attached via a laser probe holder perpendicular to the abdomen skin. The laser Doppler flow meter was set at 4 Hz at a gain of 10. The blood flow readings were taken sequentially at each site at 2-second intervals. Both ipsilateral and counterlateral data was kept and analyzed.

The raw blood flow flux measurement data was taken and the area under the curve was calculated to express results as a percentage of change in blood flow as observed from the abdomen skin innervated by ESCS for each animal. Responses were reported as a percent change from baseline blood flow values. The goals included: (1) measuring dose-dependent abdominal blood flow and vascular resistance responses during SCS with current patterns blood flow at the two sites; (2) measuring sustained blood flow (45 minutes) at a selected sub-threshold level 80-90% of motor threshold for the abdominal muscles as activated by SCS prior to and following multiple epochs of stimulation; and (3) testing timelines till the depletion of CGRP by blood flow.

### EXAMPLE 3

### Electrical Stimulation Regulates Insulin Activity in Diabetes Model Animal Systems

Obese animals and humans with or without glucose/insulin dysfunction often exhibit small sensory nerve fiber (SSNF) neuropathy. One potential mechanism is impaired transient receptor potential vanilloid 1 (TRPV1) expression on SSNFs. This study focused on identifying a corresponding SSNF neuropathy in visceral tissue (e.g., pancreas) that could be modulated by SCS. TRPV1 neuron innervation of the pancreas was investigated by measurements of: (1) vasodilatation of the abdominal skin (a surrogate for the viscera) by SCS of thoracic neural segments T9 to T11 in lean and obese rats, and rats exposed to resiniferatoxin (RTX) a capsaicin analog which depletes TRPV1 neurons and (2) morphology of the pancreas of lean, obese and RTX treated animals.

Zucker Lean (ZL), Zucker Fatty (ZF), and Sprague-Dawley (SD) rodents were implanted with a unipolar SCS electrode placed epidurally at T9 to T11 to deliver biphasic cathodal stimulation (ranges from 5 to 100 Hz pulse frequencies and 0.2 to 1 ms pulse widths for amplitudes in the low µA ranges) under general anesthesia. Current was controlled producing EMG action potentials (minimum of 100 µV peak-to-peak) in the upper abdominal muscles in order to elicit a baseline BF (neurogenic vasodilatory) response, recorded by laser Doppler flowmetry (LDF). Antidromic stimulation was used on the ZF and RTX rats. Abdominal BF was recorded at settings from 80% MT to MT. RTX-treated (250 mg/kg, sc) SD rats were studied acutely and three days after injection. Pancreatic tissue was harvested for morphological studies and confocal imaging.

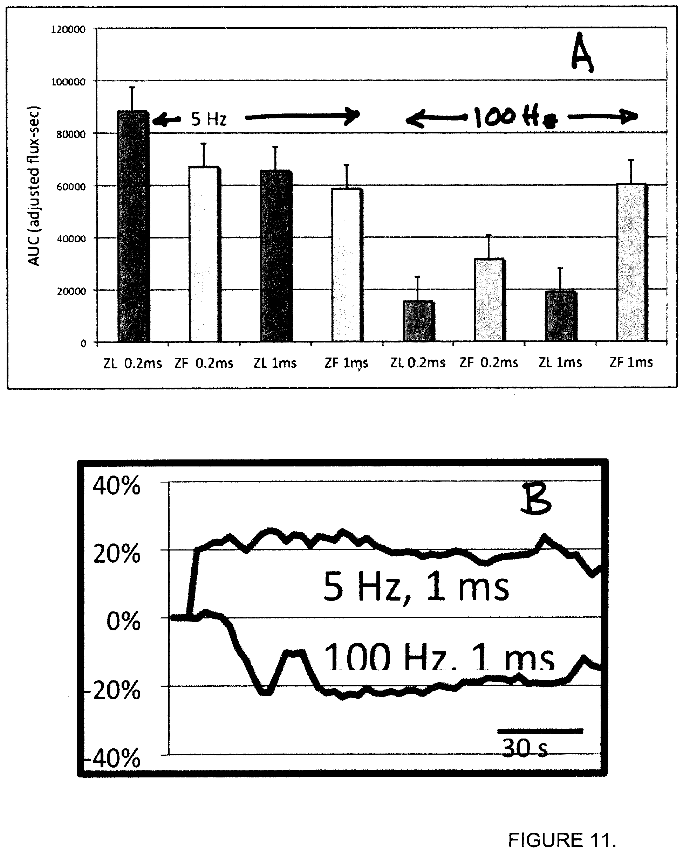

The following observations were made:
(1) SCS caused higher abdominal skin BF at 5 Hz rather than 100 Hz (Fig. 11). This can be ascribed to NA sympathetic over activity in the presence of a 100 Hz signal. A similar pattern was observed with stimulation of peripheral sensory nerves. This is further inferred by the observation that RTX-treatment did not alter negative flow;
(2) ZF rats did not exhibit negative BF suggesting impairment of sympathetic nerve fibers. The magnitude of BF in the obese rats was only minimally decreased, perhaps due to the relatively young age of the rats and/or to residual vasodilatory molecules released by SCS. Morphological studies showed marked disruption of the islets in the ZF rat with some evidence of changes in neuronal innervation;
(3) RTX-treatment deletes most of the TRPV1 neurons at the dose used in this study caused both a marked decrease in vasodilatation and a disruption of the islets. The shift to a higher threshold of current to elicit a MT and a neurogenic vasodilatation is caused by the suppressive action of RTX on both central and peripheral TRPV1 pathways; and
(4) SCS treatment elicits an insulin action from the beta cell after treatments of 45-minute duration.

### EXAMPLE 4

### Electrical Stimulation Regulates Abdominal Blood Flow

Zucker Lean (n=6) and Zucker Fatty (n=9) rats were subjected to intermediate 90 second 5 Hz (exciting) and 100 Hz (blocking) stimulation at 0.2 microseconds or one millisecond pulse durations as indicated in FIG. 11. Panel A, the four left bars are 5 Hz and the right four bars are 100 Hz. Abdominal blood flow was analyzed by a laser Doppler (Moor Instruments) measuring the area under the curve (AUC) FIG. 11. The following observations were made: (1) spinal cord stimulation caused higher abdominal skin blood flow at 5 Hz rather than 100 Hz and (2) Zucker fatty rats did not exhibit negative blood flow at inhibitory frequencies of 100 Hz suggesting that these animals do in fact have a serious impairment of sympathetic nerve fibers. The magnitude of blood flow in the obese rats was only minimally decreased, perhaps due to the relatively young age of the rats and/or to residual vasodilatory molecules released by spinal cord stimulation.

### EXAMPLE 5

### Electrical Neural Stimulation Elicits Insulin Release

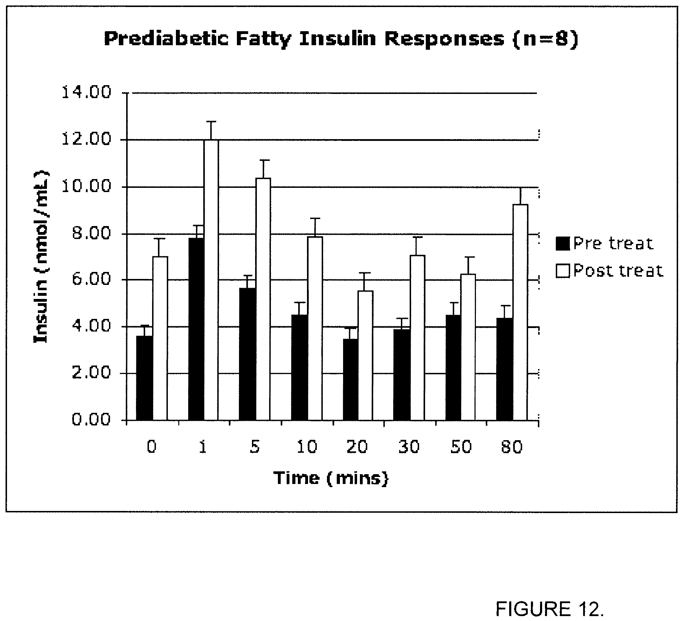

FIG. **12** depicts the results of electrical neural stimulation treatment on pre-diabetic, obese animals to elicit an insulin action response from the beta cell after treatments of a 45-minute duration with epidural spinal cord stimulation administered at dermatome regions between spinal segments T8 to T11. The average pulse duration was 250 microseconds. The average pulse frequency was 5 Hz. The average stimulation intensity to create the electric field was 100 microamperes. The Zucker Fatty (ZF) animals ranged in age from 14 to 16 weeks old and were clinically considered prediabetic based on over seven consecutive days of glucose levels over 250 mg/dL. The animals were given an intravenous glucose tolerance test (IVGTT) prior to placement of an epidural neural stimulator. The typical response curve was noted for insulin response to the glucose load of 20% concentration. The animals were treated with epidural spinal cord stimulation for a minimum of 45 minutes, at a 5 Hz frequency and pulse duration parameters of 250 microseconds. A post IVGTT was conducted. Across all animals, a marked increase in insulin release was observed, and was not observed in non-treated, control animals.

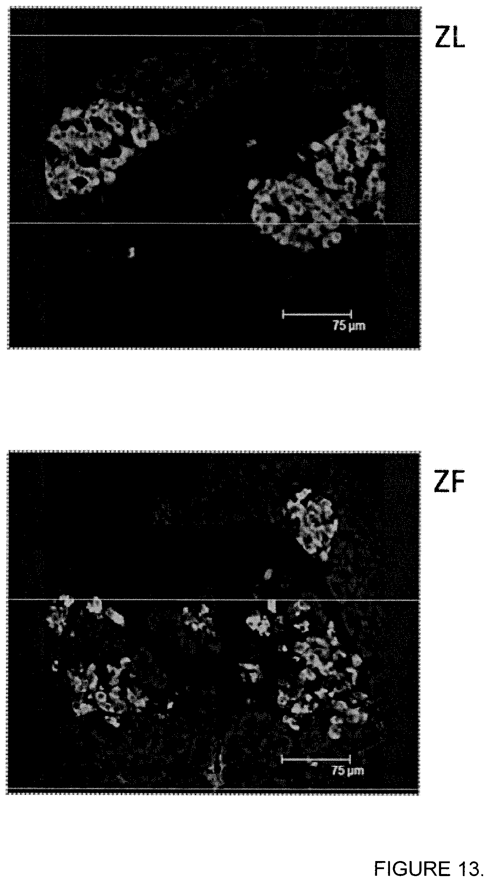

FIG. 13 shows a photomicrograph of the pancreatic islet cells of both a Zucker Lean (ZL) and Zucker Fatty (ZF) typical animal from the study. These morphological studies revealed marked disruption of the islets in the ZF rat with some evidence of changes in neuronal innervation. ZL rats demonstrated classical core/mantle Islet organization between insulin staining beta cells and glucagon staining alpha-cells. Neural innervation of the Islets revealed pronounced PGP 9.5 staining around the mantle and in/about alpha- and beta cells (Wilson 1988). Some of the PGP 9.5 stained tissue co-localize with insulin in the beta cell and with glucagon in the alpha-cell, similar to what is seen with CGRP contacts with alpha- and beta-cells (Al-Salam 2009). The pancreatic mass of a ZL rate at approximately 16 weeks is 30% larger (1.9 grams) than the average ZL rats (1.3 grams). ZF rats showed that an increased size of the Islets (many greater than 200 µm in diameter) associated with Islet distortion in shape, decreased insulin-staining with beta-cells apparently of large size, increased glucagon-staining with penetration of alpha-cells within the core, and a greater enrichment of alpha-cells about the mantle.

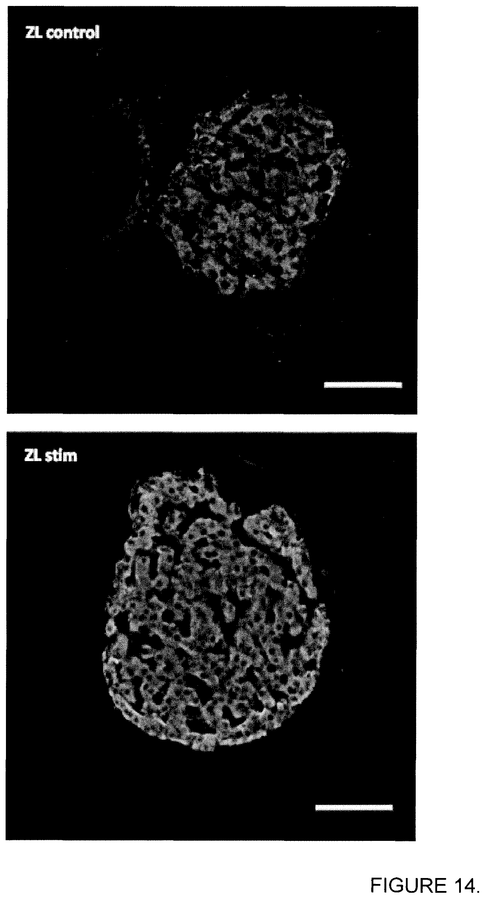

FIG. **14** shows a photomicrograph of representative islets from a Zucker Lean (ZL) control rat (top panel) and a ZL rat treated by a sequence of stimulation with an epidural spinal cord stimulator. Glucagon and PGP 9.5 staining are intensified after the 45-minute spinal cord stimulation sequence. This data suggests that the epidural stimulation parameters imposed on this animal results in greater PGP9.5 and glucagon staining in the islets of ZL rats. Green: insulin (beta cells); White: glucagon (alpha cells); Red: PGP 9.5 (neurons); Scale bar: 75 um.

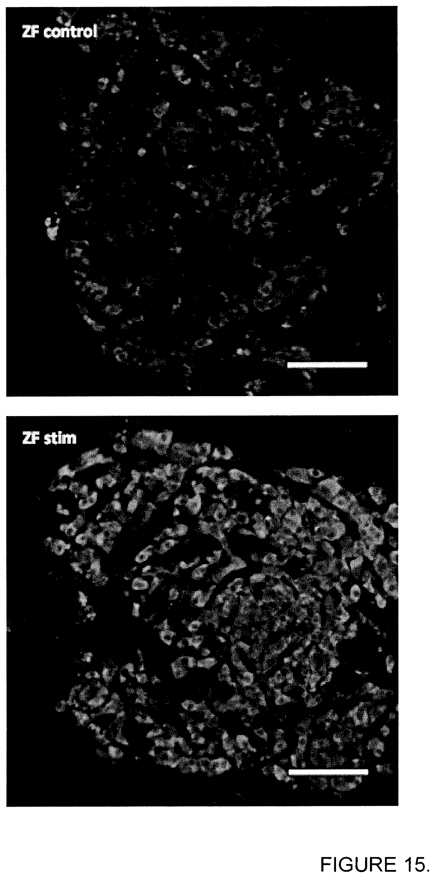

FIG. **15** shows a photomicrograph of representative islets from a Zucker Fatty (ZF) obese prediabetic rat from Charles River Labs (Boston, MA) (top) and a ZF rat treated with spinal cord stimulation from T9 to T11 at 5Hz frequency, 250 microseconds pulse width for 45 minutes. The more obvious staining for glucagon and PGP 9.5 occurs after spinal cord stimulation in the ZL animals, the healthier appearance of the islet as a whole and the more obvious staining for insulin after SCS. This data suggests that SCS results in greater PGP9.5 and glucagon staining in the islets of ZL rats and greater insulin staining in the islets of ZF pre-diabetic rats. Green: insulin (beta cells); White: glucagon (alpha cells); Red: PGP9.5 (neurons); Scale bar: 75 um.

## Claims

1. A system for modulating secretion of calcitonin gene-related peptide (CGRP) in a subject comprising:
one or more electrodes configured for positioning adjacent to or near one or more C-afferent sensory nerve fibers innervating pancreatic beta cells in a body of the subject;
a pulse generator coupled to the electrode(s) and configured to generate an electrical stimulation pulse at a frequency ranging from 60 Hz to 10,000 Hz, a pulse amplitude ranging from 0.2 to 14 volts, and a pulse width ranging from 20 µsec to 1 ms, wherein the electrical pulse is sufficient to inhibit secretion of calcitonin gene-related peptide (CGRP) from the C-afferent sensory nerve fibers,
a feedback sensor for measuring a level of a biomarker; wherein the biomarker comprises one or more of abdominal skin blood flow and abdominal skin temperature; and
a feedback controller for initiating adjustments to parameter settings of the electrical stimulation pulse if the measured biomarker level is outside a threshold range.

2. The system of claim 1 wherein the electrode(s) are positionable at or near dorsal root ganglion, splenic nerve, or dorsal column.

3. The system of claim 1 further comprising a controller configured to detect a biomarker level to at least one predetermined range, and generating electrical impulses based on the biomarker level and wherein the controller is further configured to compare the biomarker level to an historic or normative level and adjust the electrical impulse based on the comparison.

4. The system of claim 1 wherein the electrical impulse is charge-balanced.

5. An apparatus for preserving, restoring, or affecting pancreatic beta cell function in a subject, comprising:
a system in accordance with claim 1; and
an electrode lead coupled to the pulse generator and comprising one or more of said electrodes, wherein
said pulse generator is coupled to the sensor and configured to generate an electrical pulse as a function of the biomarker level.

6. The apparatus of claim 5 further comprising a radio frequency antenna for receiving incoming signals from an external programmer, wherein the radio frequency antenna is coupled to the electrode lead.

7. The apparatus of claim 5 wherein the biomarker additionally comprises one or more of insulin, glucose, CGRP, and abdominal muscle electrical activity.

8. The apparatus of claim 5 wherein the electrode lead is positionable proximal to an epidural spinal cord column at any of one or more vertebral segments from T7 to L1, dorsal root or dorsal root entry zone at any of one or more vertebral segments from T7 to L1, spinal nerve bundles leaving at any of one or more vertebral segments T7 to L1, dorsal root ganglia bundles leaving at any of one or more vertebral segments T7 to L1, peripheral nerves innervating the endocrine pancreas beta cells, abdominal nerves or their cutaneous branches, or combinations thereof.

9. The apparatus of claim 5 wherein the electrode lead comprises two or more electrode pairs and wherein the pulse generator is configured to generate multiple electrical pulses either simultaneously or sequentially.

10. The apparatus of claim 5 wherein the frequency is between about 60 and 100 Hz.

## Patentansprüche

1. System zur Modulierung der Sekretion von mit dem Calcitonin-Gen verwandtem Peptid (CGRP) bei einem Patienten, umfassend:
eine oder mehrere Elektroden, die zur Positionierung neben oder in der Nähe von einer oder mehreren C-afferenten Sinnesnervenfasern, die Pankreas-Betazellen in einem Körper des Patienten anregen, ausgelegt sind;
einen Impulsgenerator, der an die Elektrode(n) gekoppelt und ausgelegt ist, um einen elektrischen Stimulationsimpuls bei einer Frequenz, die von 60 Hz bis 10.000 Hz reicht, einer Impulsamplitude, die von 0,2 bis 14 Volt reicht, und einer Impulsbreite, die von 20 µsec bis 1 ms reicht, zu erzeugen, wobei der elektrische Impuls ausreichend ist, um die Sekretion von mit dem Calcitonin-Gen verwandtem Peptid (CGRP) aus den C-afferenten Sinnesnervenfasern zu hemmen,
einen Feedback-Sensor zum Messen eines Spiegels eines Biomarkers; wobei der Biomarker eines oder mehrere von abdominalem Hautblutfluss und abdominaler Hauttemperatur umfasst; und
eine Feedback-Steuerung zum Initiieren von Anpassungen an Parametereinstellungen des elektrischen Stimulationsimpulses, wenn der gemessene Biomarkerspiegel außerhalb einer Schwellenspanne liegt.

2. System nach Anspruch 1, wobei die Elektrode (n) an oder in der Nähe von Spinalganglien, Milznerv oder Dorsalsäule positionierbar sind.

3. System nach Anspruch 1, ferner umfassend eine Steuerung, die ausgelegt ist, um einen Biomarkerspiegel bis zumindest einer zuvor festgelegten Spanne zu erfassen und die elektrische Impulse auf Grundlage des Biomarkerspiegels erzeugt und wobei die Steuerung ferner ausgelegt ist, um den Biomarkerspiegel mit einem historischen oder normativen Spiegel zu vergleichen und den elektrischen Impuls auf Grundlage des Vergleichs anzupassen.

4. System nach Anspruch 1, wobei der elektrische Impuls ladungsausgeglichen ist.

5. Vorrichtung zur Erhaltung, Wiederherstellung oder Beeinflussung der Pankreas-Betazellenfunktion bei einem Patienten, umfassend:
ein System nach Anspruch 1; und
eine Elektrodenleitung, die an den Impulsgenerator gekoppelt ist und eine oder mehrere der Elektroden umfasst, wobei der Impulsgenerator an den Sensor gekoppelt und ausgelegt ist, um einen elektrischen Impuls in Abhängigkeit des Biomarkerspiegels zu erzeugen.

6. Vorrichtung nach Anspruch 5, ferner umfassend eine Funkfrequenzantenne zum Empfangen von eingehenden Signalen von einem externen Programmierer, wobei die Funkfrequenzantenne an die Elektrodenleitung gekoppelt ist.

7. Vorrichtung nach Anspruch 5, wobei der Biomarker zusätzlich eines oder mehrere von Insulin, Glucose, CGRP und abdominaler elektrischer Muskelaktivität umfasst.

8. Vorrichtung nach Anspruch 5, wobei die Elektrodenleitung proximal zu einer epiduralen Rückenmarkssäule an einem beliebigen oder mehreren vertebralen Segmenten von T7 bis L1, einem dorsalen Wurzel- oder dorsalen Wurzeleingangsbereich an einem beliebigen oder mehreren vertebralen Segmenten von T7 zu L1, Spinalnervbündeln ausgehend an einem beliebigen oder mehreren vertebralen Segmenten T7 bis L1, dorsalen Wurzelganglienbündeln ausgehend an einem beliebigen oder mehreren vertebralen Segmenten T7 bis L1, peripheren Nerven, welche die endokrinen Pankreas-Betazellen anregen, abdominalen Nerven oder ihren kutanen Zweigen oder Kombinationen davon positionierbar ist.

9. Vorrichtung nach Anspruch 5, wobei die Elektrodenleitung zwei oder mehr Elektrodenpaare umfasst und wobei der Impulsgenerator ausgelegt ist, um mehrere elektrische Impulse entweder simultan oder sequentiell zu erzeugen.

10. Vorrichtung nach Anspruch 5, wobei die Frequenz zwischen ungefähr 60 und 100 Hz liegt.

## Revendications

1. Système pour moduler la sécrétion du peptide lié au gène de la calcitonine (CGRP) chez un sujet comprenant :
une ou plusieurs électrodes conçues pour se positionner de manière adjacente à ou à proximité d'une ou de plusieurs fibres nerveuses sensorielles C-afférentes innervant des cellules bêta pancréatiques dans le corps du sujet ;
un générateur d'impulsions couplé à/aux (l')électrode(s) et conçu pour générer une impulsion de stimulation électrique à une fréquence allant de 60 Hz à 10 000 Hz, une amplitude d'impulsion allant de 0,2 à 14 volts et une largeur d'impulsion allant de 20 µsec à 1 ms, dans lequel l'impulsion électrique est suffisante pour inhiber la sécrétion du peptide lié au gène de la calcitonine (CGRP) à partir des fibres nerveuses sensorielles C-afférentes ;
un capteur de rétroaction pour mesurer un niveau d'un biomarqueur ; dans lequel le biomarqueur comprend un ou plusieurs éléments parmi un flux sanguin de la peau abdominale et la température de la peau abdominale ; et
un dispositif de commande de rétroaction pour initier des ajustements aux réglages de paramètres de l'impulsion de stimulation électrique si le niveau de biomarqueur mesuré est en dehors d'une plage de seuil.

2. Système selon la revendication 1, dans lequel (l')/les électrode(s) peut/peuvent être positionnée (s) sur ou à proximité du ganglion de la racine dorsale, du nerf splénique ou de la colonne dorsale.

3. Système selon la revendication 1, comprenant en outre un dispositif de commande conçu pour détecter un niveau de biomarqueur à au moins une plage prédéterminée, et générant des impulsions électriques sur la base du niveau de biomarqueur et dans lequel le dispositif de commande est en outre conçu pour comparer le niveau de biomarqueur à un niveau historique ou normatif et ajuster l'impulsion électrique sur la base de la comparaison.

4. Système selon la revendication 1, dans lequel l'impulsion électrique est équilibrée en charge.

5. Appareil pour préserver, restaurer ou affecter la fonction des cellules bêta pancréatiques chez un sujet, comprenant :
un système selon la revendication 1 ; et
un conducteur d'électrode couplé au générateur d'impulsions et comprenant une ou plusieurs desdites électrodes, dans lequel ledit générateur d'impulsions est couplé au capteur et conçu pour générer une impulsion électrique en fonction du niveau de biomarqueur.

6. Appareil selon la revendication 5, comprenant en outre une antenne radiofréquence pour recevoir des signaux entrants d'un programmateur externe, dans lequel l'antenne radiofréquence est couplée au conducteur d'électrode.

7. Appareil selon la revendication 5, dans lequel le biomarqueur comprend en outre un ou plusieurs éléments parmi l'insuline, le glucose, le CGRP et l'activité électrique du muscle abdominal.

8. Appareil selon la revendication 5, dans lequel le conducteur d'électrode est positionnable à proximité d'une colonne de la moelle épinière épidurale au niveau de l'un quelconque d'un ou de plusieurs segments vertébraux T7 à L1, de la racine dorsale ou de la zone d'entrée de la racine dorsale au niveau de l'un quelconque d'un ou de plusieurs segments vertébraux T7 à L1, des faisceaux de nerfs rachidiens sortant au niveau de l'un quelconque d'un ou de plusieurs segments vertébraux T7 à L1, des ganglions spinaux de la racine dorsale sortant au niveau de l'un quelconque d'un ou de plusieurs segments vertébraux T7 à L1, des nerfs périphériques innervant les cellules bêta pancréatiques endocrines, les nerfs abdominaux ou leurs branches cutanées, ou des combinaisons de ceux-ci.

9. Appareil selon la revendication 5, dans lequel le conducteur d'électrode comprend deux paires d'électrodes ou plus et dans lequel le générateur d'impulsions est conçu pour générer plusieurs impulsions électriques simultanément ou séquentiellement.

10. Appareil selon la revendication 5, dans lequel la fréquence est comprise entre environ 60 et 100 Hz.
